# EUROPEAN PATENT SPECIFICATION

(11) **EP 0 568 647 B1**
(45) Date of publication and mention of the grant of the patent: **11.09.1996**
(21) Application number: 92906222.2
(22) Date of filing: 17.01.1992
(51) Int. Cl.: C12N 9/24, C12P 21/00, C12N 1/38, C12N 5/00

(54) **PRODUCTION OF ENZYMATICALLY ACTIVE GLUCOCEREBROSIDASE FROM RECOMBINANT CELLS**
HERSTELLUNG ENZYMATISCHER AKTIVER GLUKOCEREBROSIDASE VON REKOMBINANTEN ZELLEN
GLUCOCEREBROSIDASE A ACTION ENZYMATIQUE A PARTIR DE CELLULES RECOMBINEES

(30) Priority: 21.01.1991 US 644159
(43) Date of publication of application: 10.11.1993
(73) Proprietor: GENZYME CORPORATION, Cambridge, Massachusetts 02139-1562 (US)
(72) Inventor: HAYES, Michael, L., Acton, MA 01720 (US); BARNGROVER, Debra, A., Wayland, MA 01778 (US); RASMUSSEN, James, R., Cambridge, MA 02142 (US)
(74) Representative: Overbury, Richard Douglas
(86) International application number: US9200431
(87) International publication number: WO9213067

(56) References cited:
- WO-A-88/00967
- WO-A-90/07573
- US-A- 3 910 822

## Description

### Technical Field

The invention relates to the production of proteins (such as glucocerebrosidase) in cell cultures using recombinant techniques, and in particular to techniques permitting high levels of expression and secretion of the proteins into stabilized environments.

### Background Art

Gaucher's disease is a genetic disorder which has been shown to be due to a deficiency of glucocerebroside-β-glucosidase (GCR). In the most common form of the disease (Type I) this results in an accumulation of glucocerebroside in phagocytic cells of mainly liver, spleen and bone marrow of the afflicted patients. Brady, R. O. et al. (1966) J. Clin. Invest. 45 pp. 1112-1115. GCR can be isolated from human placental tissue. Pentchev and Brady (1975) U.S. Patent No. 3,910,822. However, the supply of human placental tissue is ultimately limited, which may limit the number of patients which could be treated with enzyme from this source.

The gene for GCR has been cloned. Sorge, J. et al. (1985) Proc. Natl. Acad. Sci., USA 82 pp. 1289-7293 and (1986) Proc. Natl. Acad. Sci., USA 83 pp. 3567; and Ginns U.S. Pat. Appl. Serial No. 137796, filed 12/23/87. This could theoretically allow for unlimited production of the enzyme, using recombinant cells grown in large quantities. Methods for high level production of proteins in mammalian cells have been reviewed. Kaufman, R. J. (1987) Genetic Engineering 9 pp. 155-198 (J. Setlow, ed.) Plenum Press, New York. Production of vaccines using microcarriers and mammalian cell culture has been reported. Van Wezel, A. L. and van der Velden-de Groot, C. A. M. (1978) Process Biochemistry, March pp. 6-8. The cells were grown on microcarriers in cell culture medium and the culture was mechanically stirred and sparged to provide the necessary oxygen. For the production of rabies vaccine, the growth medium was removed and the cell covered microcarriers were resuspended in viral culture medium. Multiple harvests were obtained by harvesting the viral-containing medium and replacing with fresh medium every 4 to 5 days. Production of a secreted recombinant protein by a mammalian cell culture was reported by using a culture medium for growth of cells, and then a second medium containing a non-toxic polymer, which acts as a cell protective agent, during protein production; the production medium is continually removed and replaced for continuous production of protein. Gray, P. P. et al. (1988) WO Patent No. 8800967.

There are two potential problems which could limit the cell culture production of GCR. The first is that the enzyme is normally not secreted, but is instead directed to the lysosome, an intracellular organelle. Since there are physiological limitations on the amount of intracellular protein a cell can contain, this can constitute an obstacle to the development of a cost-effective process. The second potential problem is that GCR is unstable at the 37°C required for mammalian cell culture in either phosphate-buffered saline (PBS) or PBS + 1mM dithiothreitol (DTT). Humphreys, J. D. and Ihler, G. J. (1980) J. Lab. Clin. Med. 96 pp. 682-692. If the enzyme were secreted by mammalian cells, methods would need to be found to stabilize its activity at 37°C. The methods selected would have to be compatible with maintaining high cell viability and productivity and not unduly interfere with the eventual purification of the enzyme from the extracellular medium. High levels of glycerol (20-50%) have been used for stabilizing the placental enzyme during its purification (Pentchev and Brady, op. cit.) but this method would be unacceptable for use with mammalian cell culture because high levels of glycerol are toxic to mammalian cells. High levels of bovine serum albumin (100 mg/ml) (Humphreys and Ihler, op. cit.) are known to stabilize the protein but would interfere with the purification of the protein. It has also been shown that the stability of free GCR is enhanced by entrapment in hemolyzed resealed erythrocytes, but this would also interfere with the purification. Humphreys and Ihler, op. cit.

The reducing composition dithiothreitol (DTT) is commonly used as a stabilizing agent during the purification of intracellular proteins, usually at a pH of >7.5, since the reaction rate is faster at an alkaline pH. Creighton, T.E. (1984) Methods in Enzymology 107 pp. 305-329. DTT, however, reduces cystine to cysteine, and thus proteins with disulfide links would not be expected to be stabilized by DTT. For example, the presence of DTT during enzyme isolation was found to inactivate α-fetoprotein. Wu, J. T. et al. Abstract: XVth Annual Meeting ISOBM. The presence of DTT has also been found to be toxic to cells in cell culture. For example, the use of DTT in the culture of cystinotic fibroblasts showed that DTT could be used at 0.1 mM; however, higher levels (>1.0 mM) were found to be toxic to the cells due to the stimulation of respiration. Goldman et al. (1970) Lancet pp. 811. Bettger, et al. reported the use of DTT in a serum-free medium for growth of human diploid fibroblasts, at a very low level of 6.5 X 10⁻³ mM and found the benefit to be only marginal. Bettger, W. J. et al. (1981) Proc. Nat. Acad. Sci. USA 78 pp. 5588-5592. Trivedi, et al. reported the use of 1 mM DTT in hepatocyte cultures to enhance the reduction of Vitamin K 2,3-epoxide and found that exposure to the DTT did not affect the protein concentration of the cells, however the cells were never cultured for longer than 48 hours with DTT. Trivedi, L. S. et al. (1988) Arch. Biochem. Biophys. 264 pp. 67-73. DTT at 20 mM has been used to enhance the survival of cells exposed to radiation, however the cells were exposed to DTT for less than one hour, and the protective effect of the DTT was thought to be due to the depletion of culture oxygen, a condition which would not be compatible with long-term viability of the cells. Biaglow, J. E. (1982) Advances in Exp. Medicine and Biology, Vol. 157 Hyperthermia, Dicher, H. I. and Bruley, D. F., eds., Plenum Press, NY, pp. 147-175. Although Pentchev has found that DTT may be used to stabilize GCR in the last (cell-free) steps of enzyme purification (Pentchev and Brady, op. cit.), the foregoing references suggest that use of DTT in effective concentrations (≥ 1.0 mM) would threaten the viability of cell cultures used to produce GCR.

Several investigators have examined the relationship between concentration of dissolved oxygen in a hybridoma cell culture and productivity of the hybridoma cells in producing antibody. Reuveny, et al. found that lowering the oxygen concentration to 25% of air saturation (corresponding to 0.05 mM) increased monoclonal antibody yield by prolonging cell viability. Reuveny, S. et al (1986) J. Immunolog. Meth. 86 pp. 53-59. The effect seems to depend on the hybridoma line studied, however, since Miller, et al. studied oxygen concentrations between 0.1 to 100% air saturation and found the optimal concentration of oxygen for antibody production to be 50% (0.1 mH), while MacMichael found that productivity decreased at oxygen concentrations below 2.48 mg/l (0.08 mM). Miller, W. M. et al. (1987) J Cell. Physiol. 132 pp. 524-530. MacMichael, G. J. (1989) Amer. Biotech. Lab. January, pp. 44-47. In all the work cited, the effect of oxygen was to increase the population of viable cells, thus increasing the amount of antibody produced, rather than enhancing the stability of the antibody. The effect of pH on hybridoma productivity has also been examined. MacMichael investigated the range of 6.8 to 8.1 and found the maximal productivity to be between 7.2 and 7.4. MacMichael, op. cit. Other investigators found the optimal pH to be 6.8-6.9 for a different hybridoma line. Maiorella, B., et al. (1989) ACS National Fall Meeting, Miami. In both cases, the pH change was assumed to change the specific productivity of the cell lines, rather than to stabilize the protein. The optimal pH for cell culture is considered to be 7.4 and that this optimum varies little (between 7.0 and 7.7) for normal and transformed cell lines. Freshney, R.I. (1987) Culture of Animal Cells, A Manual of Basic Technique, Alan R. Liss, Inc. New York, p. 69.

According to one aspect of the present invention there is provided a method of producing biologically active glucocerebrosidase comprising:
(i) culturing recombinant cells containing the DNA sequence encoding for the production of glucocerebrosidase in a growth medium suitable for cell growth;
(ii) replacing a substantial portion of the growth medium with a production medium that is suitable for cell viability and secretion and stabilization of glucocerebrosidase;
(iii) allowing production and secretion of glucocerebrosidase into the production medium;
(iv) removing and collecting the production medium containing the secreted glucocerebrosidase, obtained in step (iii); and
(v) purifying the glucocerebrosidase from the production medium collected in step (iv).

A wide range of compositions, that are not toxic to the cells, may be added to the production medium to stabilize GCR. Compositions containing thiols may be added, such as dithiothreitol, glutathione, 1,3-mercaptoglycerol, cysteamine, lipoic acid, lipoamide and thioglycolate or proteins such as serum or serum albumin may be added. The pH or dissolved oxygen concentration may be adjusted, or a combination of such stabilizing conditions may be utilized.

According to another aspect of the present invention there is provided a method for the production and stabilization of a protein comprising:
(i) culturing recombinant cells containing the DNA sequence encoding for the production of the protein in a growth medium suitable for cell growth;
(ii) replacing a substantial portion of the growth medium with a production medium, suitable for cell viability and secretion and stabilization of the protein, that includes dithiothreitol in the range of approximately 0.5-2 mM and is maintained at a less than 0.06 mM concentration of molecular oxygen and at a pH adjusted to below 7;
(iii) allowing production and secretion of the protein into the production medium;
(iv) removing and collecting the production medium containing the secreted protein, obtained in step (iii), on a continuous basis and continually replacing the production medium; and
(v) purifying the protein from the production medium collected in step (iv).

According to a further aspect of the present invention there is provided a cell culture medium comprising:
(i) cells including means for secreting glucocerebrosidase; and
(ii) a composition that is not toxic to the cells and that stabilizes glucoerebrosidase.

### Brief Description of the Drawings

The foregoing features of invention will be more readily understood by reference to the following detailed description taken with the accompanying drawings, in which:

Fig. 1 is a graph illustrating of the effect of donor calf serum levels on the medium levels of r-GCR, in accordance with Example 1.

Fig. 2 is a graph of medium levels of rGCR vs pH with 1%, 5%, and 10% concentrations of DCS, in accordance with Example 1.

Fig. 3 is a graph of medium levels of rGCR vs. dissolved oxygen concentration with 1%, 5%, and 10% concentrations of DCS, in accordance with Example 1.

Fig. 4 is a graph showing the effect of DTT on GCR production, in accordance with Example 2.

Fig. 5 is a graph of medium levels of r-GCT vs. pH with 0 to 0.5 mM DTT as described in Example 2.

Fig. 6 is a graph showing the effect of 1mM DTT on the production and specific activity of rGCR, in accordance with Example 3.

Fig. 7 is a graph illustrating the effect of BSA on GCR production as described in Example 4.

Fig. 8 is a graph of medium levels of rGCR vs. pH with BSA in accordance with Example 4.

Fig. 9 is a graph illustrating the effect of pH on the concentration and specific activity of GCR in accordance with Example 5.

Fig. 10 is a graph comparing concentration and specific activity of GCR in two cell lines in accordance with Example 6.

### Detailed Description of Specific Embodiments

### General

The invention provides a method for production of a protein, such as glucocerebrosidase (GCR), wherein recombinant cells containing the DNA sequence encoding for the production of the protein in a cell culture are grown in a growth medium suitable for cell growth.

After growth of the cell culture, a substantial portion of the growth medium is removed and replaced with a production medium adequate for cell viability and suitable for secretion and stabilization of the protein. Upon expression and secretion of the protein into the production medium, the production medium is removed and collected. The protein is then purified from the production medium. Alternatively, a substantial portion of the production medium may be continually removed, collected and replaced to allow continuous secretion and production of protein. The collected protein may then be purified by the processing or purification steps required to obtain pure protein.

Compositions which stabilize the protein may be added to the production medium. Compositions which may be added include thiol-containing dompounds such as dithiothreitol, glutathione, 1,3-mercaptoglycerol, cysteamine, lipoic acid, lipoamide and thioglycolate or proteins such as serum or serum albumin may be added. The pH or dissolved oxygen concentration may be adjusted, or a combination of stabilizing conditions may be utilized.

### Glucocerebrosidase

The method of the invention may be applied to the large scale production of glucocerebrosidase (GCR) from mammalian cell culture. The invention may thereby provide a source for large scale quantities of purified GCR, which is used in replacement therapy in the treatment of Gaucher's disease.

Recombinant cells producing GCR may be developed by a series of steps which are well-known in the field, which include construction of a plasmid containing the DNA encoding GCR, various enhancers and plasmid replication elements and possibly selective markers or amplifiable genes, followed by transfecting the plasmid into a suitable mammalian host cell. The cell lines which most efficiently express protein may be selected by expressing the protein in a selective medium, and examining the levels of intracellular GCR protein or mRNA. The cellular productivity may be amplified by step-wise increases in the selective agent if an amplifiable marker is included in the plasmid. The particular form of expression system used to produce GCR forms no part of the present invention.

Once the recombinant cells have been developed, they may be cultured by a number of techniques which are well-known in the field, which include, but are not limited to, suspension, microcarrier, roller bottle, or hollow fiber cultures. Preferably, the cells are grown on microcarriers which allow for easy separation of the medium from the cells and which can be scaled up to large volumes. The microcarriers used must support the growth of the cells and can be one of several known to the field, such as collagen coated dextran, DEAE-substituted dextran, gelatin or polystyrene. The microcarriers are best maintained in suspension, preferably with the use of a low shear agitation method such as 250 ml spinner flasks (Bellco) which have a suspended magnetic stir bar and a teflon paddle.

During the growth phase, regular exchanges of the medium can be performed to replace the consumed nutrients and remove the generated waste products. This can easily be done by stopping the agitation, allowing the microcarriers to settle, removing 80% of the culture medium and replacing it with the same amount of fresh growth medium. The growth medium used can be any of the several which are well-known in the field and which can contain animal serum or other growth-promoting mixtures. Preferably, this growth medium will allow the cells to reach a high density in the reactor (>5 x 10⁶ cells/ml of medium used). Once a suitable, preferably high cell density is achieved, the growth medium is replaced with a production medium that contains compounds which stabilize the secreted GCR and which maintain the cells in a viable, slow growing or non-growing productive state. The concentrations of the additives used must be such as to allow for maintenance of cell viability and productivity, while not unduly interfering with later purification. Since the cells are maintained in a stationary, slow growing or non-growing state, higher levels of additives, which might normally interfere with the growth of the cells, can be used.

Thiol titrations using dithionitrobenzoate showed the presence of at least one thiol on glucocerebrosidase required for enzyme activity. This indicated that conditions which stabilize thiols might stabilize GCR in cell culture. In accordance with this observation, it was found that conditions which stabilize thiols; such as the addition of a thiol-containing composition, or reducing the concentration of dissolved oxygen, or modifying the pH, stabilize the activity of GCR in cell culture.

In the production medium, for optimal stabilization of GCR, the preferred level of dithithreitol (DTT) is 0.5-2 mM, while the optimal pH of the production medium may be below 7.0 and preferably below 6.6. The lower limit of pH is determined by the pH sensitivity of the particular cells used. The pH used here is believed to be novel because it is lower than that commonly used in cell culture. The dissolved oxygen concentration must be kept low, preferably below 0.06 mM. Normally, the reductive capacity of DTT is enhanced by a high pH and a pH of 8.1 has been recommended. Cleland, W. W. (1964) Biochemistry 3 pp. 480. however, for this process, in the production of GCR, the stabilizing effect of DTT is enhanced by a lower pH. Other reducing compositions which may be added to the production medium to stabilize the activity of GCR include thiols such as glutathione, 1,3-mercaptoglycerol, cysteamine, lipoic acid, lipoamide and thioglycolate or proteins such as serum or serum albumin.

If the cells have been cultured by a method which allows easy separation of cells and medium (such as with microcarrier or hollow fiber culture), then this separation can be performed ona regular basis and the conditioned medium containing the product can be removed and replaced with fresh production medium. Alternatively, this separation can be performed on a continuous basis in a perfusion mode.

If suspension culture is used, then the cells and medium can be removed from the reactor, and separated by some appropriate method, such as continuous centrifugation, centrifugation or hollow fibers. The cells can then be rediluted with fresh maintenance medium and returned to the reactor for continued production if desired.

The invention may be used for producing large scale quantities of GCR for clinical use in the treatment of Gaucher's disease. In addition, the invention may be applied to other proteins.

### EXAMPLES

### Example 1.

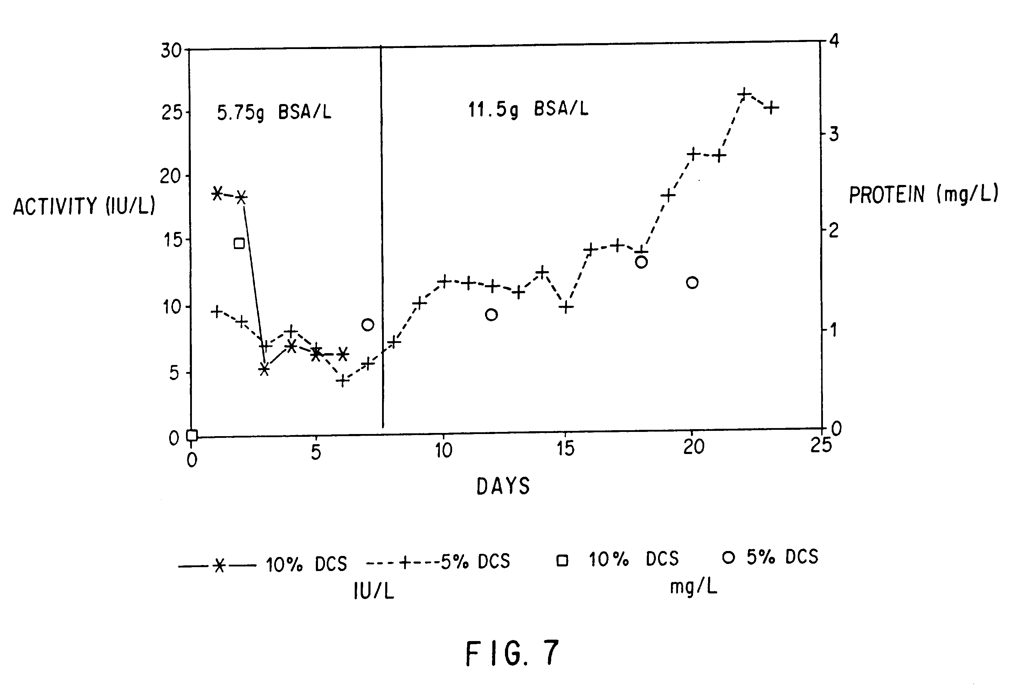

GCR expression vectors were constructed with DNA sequences coding for glucocerebrosidase, under control of the SV40 promoter, the adenovirus promoter, or the RSV-LTR promoter, together with an amplifiable gene such as dihydrofolate reductase, adenosine deaminase, or ornithine decarboxylase, or others, such that the amplifiable gene is expressed under the control of its own promoter, or linked to the GCR gene, by techniques obvious to those skilled in the art, and as has been reviewed by Kaufman, R. J. (1987) Genetic Engineering 9 pp. 155-198. In one example, a GCR expression vector was constructed with the gene encoding GCR under the control of the SV40 enhancer early promoter as described in U.S. Patent Appl. No. 455,507, filed December 22, 1989 (Fig. 7). The GCR vector was then transfected into Chinese hamster dihydrofolate reductase deficient mutant cells DG44 (G. Urlaub et al. (1986) Som. Cell Molec. Genet. 12 pp. 555-666), using the lipofectin™ transfection method as described by the manufacturer (BRL, Gaithersburg, MD). After transfection, the transfecting medium was removed and replaced with a non-selective medium for 24 hours. This medium was then replaced with a selective medium, α-MEM (minus nucleotides). Cell colonies which grew in the selective medium were harvested and aliquots lysed with a 0.05M sodium citrate buffer, pH 6.2, containing 12.0 g/L sodium cholic acid and 12.0 ml/L of 1-butanol. The intracellular GCR levels were measured in the lysed samples using the fluorogenic substrate 4-methyl-umbelliferyl-β-D-glucoside. Suzuki, K. Methods in Enzymol. (1978) 50 pp. 478-479. Cell colonies containing high levels of intracellular GCR were expanded and stepwise amplified with increasing levels of methotrexate up to 10.0 mM.

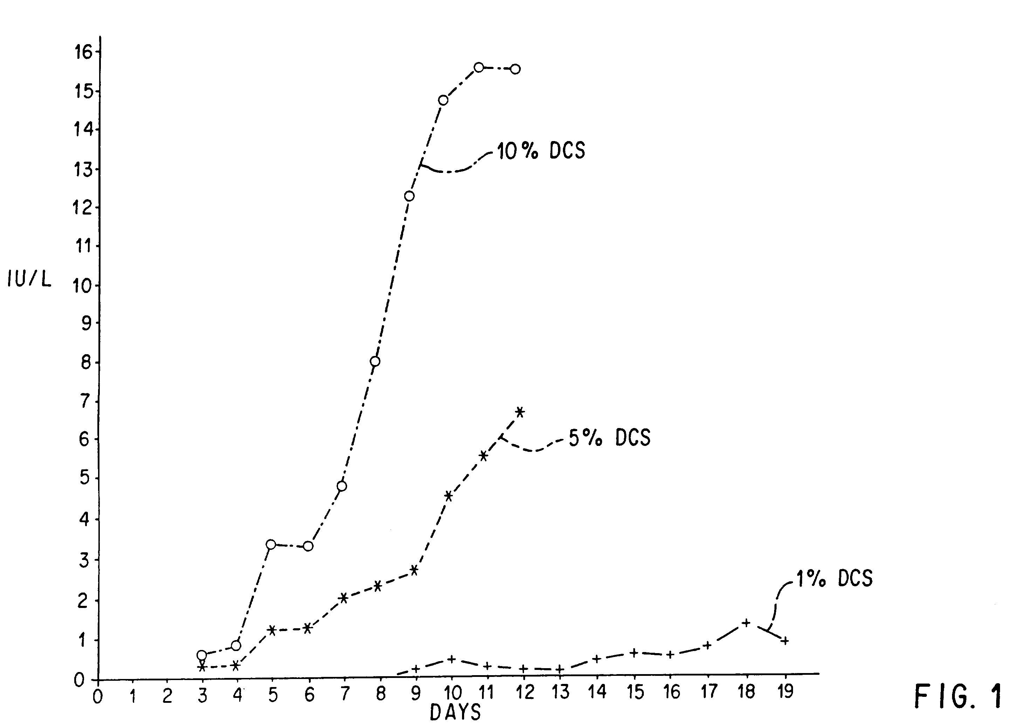

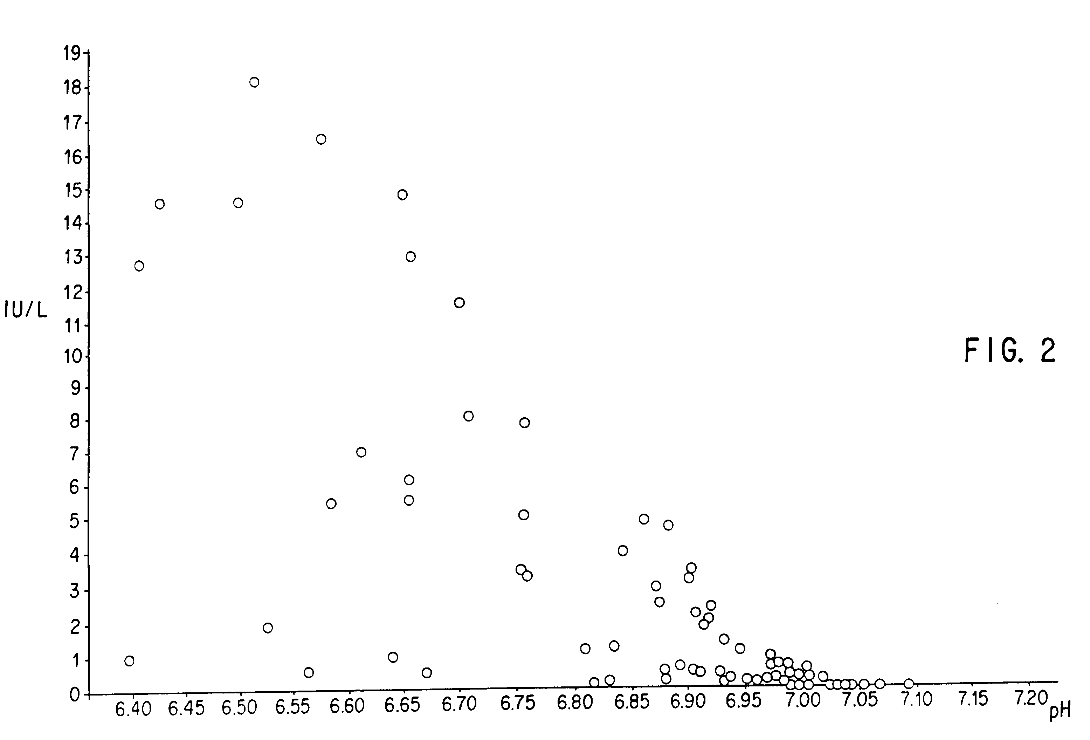

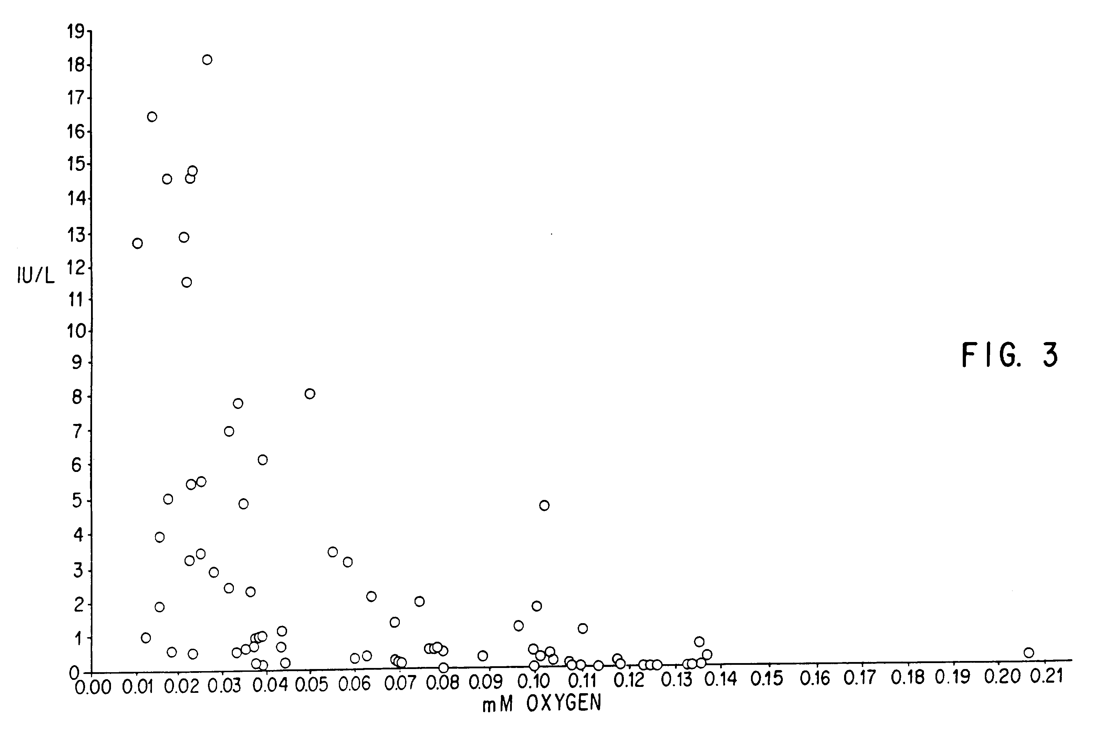

One cell line, II 15B, was taken at this point and inoculated into microcarrier culture at an initial density of 1 X 10⁵ cells/ml, in a growth medium consisting of basal medium plus 1%, 5% or 10% donor calf serum (DCS). For this example, 80% of the medium was exchanged starting on day 3 and every 48 hours after that. The units of GCR activity present in the extracellular medium, and the pH and dissolved oxygen concentration of the medium were monitored in daily samples. Cell growth rates were similar between the cultures given 10% or 5% serum. Higher levels of GCR activity (as measured by the 4-MU assay) in the extracellular medium were found with higher levels of serum (Fig. 1), lower pH (Fig. 2) and lower dissolved oxygen (Fig. 3). Specifically, the desired pH is below 6.8 and for these cells, preferably between 6.4 and 6.6 while the desired dissolved oxygen concentration is below 0.06 mM.

### Example 2.

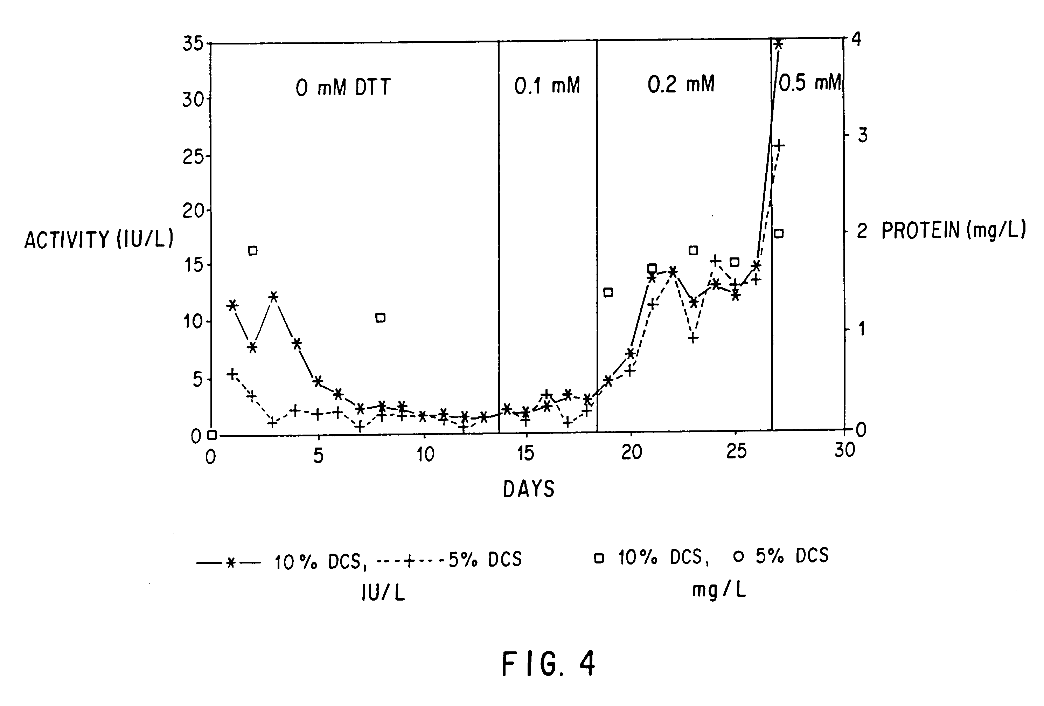

The II 15B cell line used in example 1 was inoculated into microcarrier spinners, using the same methods as in example 1 and a growth medium consisting of a basal medium plus either 5% or 10% DCS. After the cells reached a density of 8 x 10⁶ cells/ml, the production phase was then initiated by removing the growth medium and replacing it with the basal medium alone. The medium was exchanged (80%) every 24 hours and gradually increasing levels (0 to 0.5 mM) of dithiothreitol were added to the basal medium. The dissolved oxygen was maintained below 0.06 mM by controlling the level of oxygen in the headspace of the reactor. The level of enzyme activity (measured by the 4-MU assay) found in the extracellular medium as a function of increasing amounts of DTT is shown in Fig. 4.

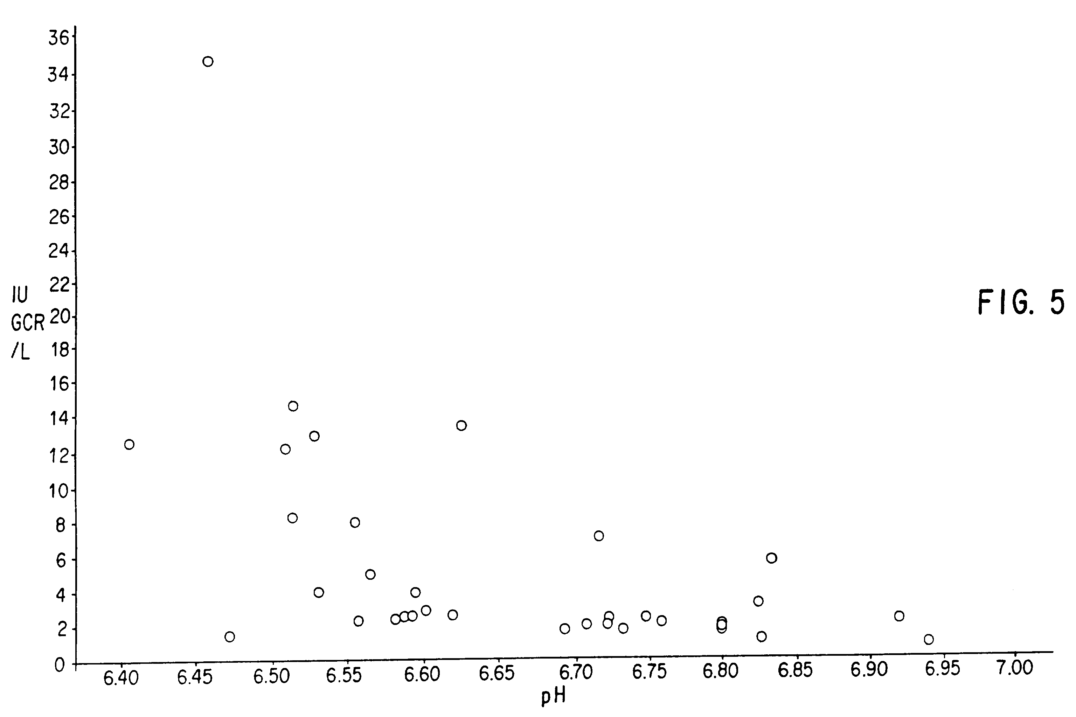

Selected samples were also analyzed for total GCR protein by a polyclonal ELISA and demonstrated a steady level of secretion of GCR protein of 1.64 ± 0.25 mg/l. Thus the increased level of enzyme activity measured in the medium represented increased stability of the secreted enzyme. This increased activity was not only correlated with increased levels of DTT, but also with decreased pH, as demonstrated in Fig. 5. The preferred pH for high enzyme activity was below 6.6.

### Example 3.

The II 15B cell line used in Example 1 was inoculated into a separate set of microcarrier spinners, using the same methods as in example 1 and a growth medium consisting of a basal medium plus 10% DCS. After the cells reached a density of 1 x 10⁷ cells/ml, the cultures were switched to a production medium containing 1.0 mM DTT. During the production period, the pH was always maintained below 6.8 and generally below 6.6. The dissolved oxygen was maintained below 0.06 mM. An 80% harvest and refeed was performed daily and the levels of GCR in the extracellular medium were monitored in daily samples by both the 4-MU activity assay and a polyclonal ELISA.

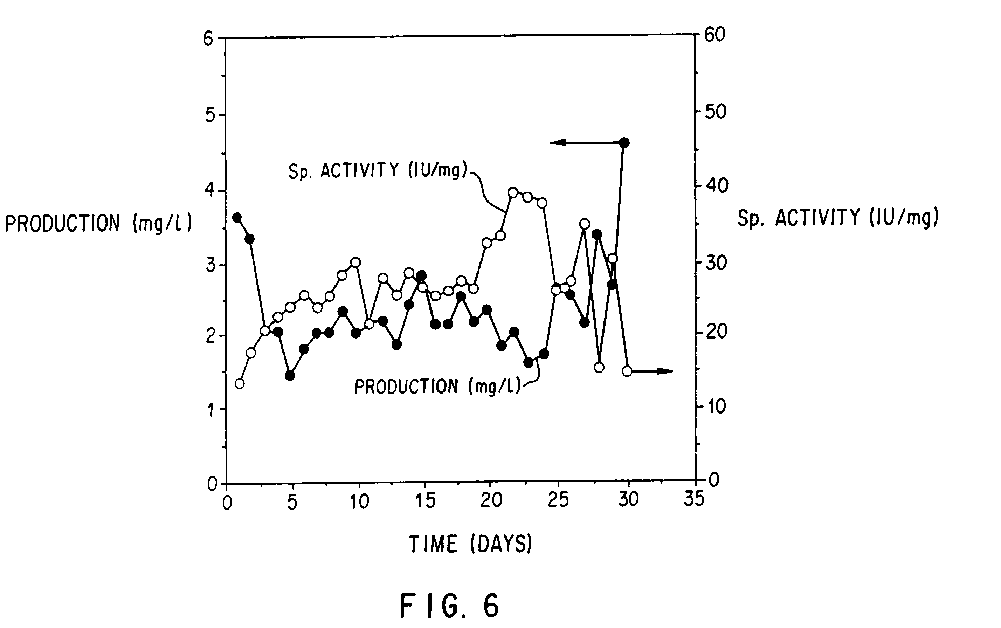

Figure 6 shows that productivity of the cells could be maintained for 30 days and that the specific activity of GCR in the extracellular medium was generally above 25 IU/mg. Ten liters of harvest medium from these cultures were purified with two chromatography steps with a total yield of 22 mg of protein (BCA) that had a specific activity of 42 IU/mg of protein.

### Example 4.

The II 15B cell line used in Example 1 was inoculated into a separate set of microcarrier spinners, using the same methods and growth medium as in Example 1. After the cells reached a density of 8 x 10⁶ cells/ml the cultures were switched to a production medium containing 5.75 g/l bovine serum albumin. The level of albumin was increased to 11.5 g/l after 7 days. The level of active enzyme, pH and dissolved oxygen were again monitored in daily samples. The dissolved oxygen was maintained below 0.06 mM as in Example 1.

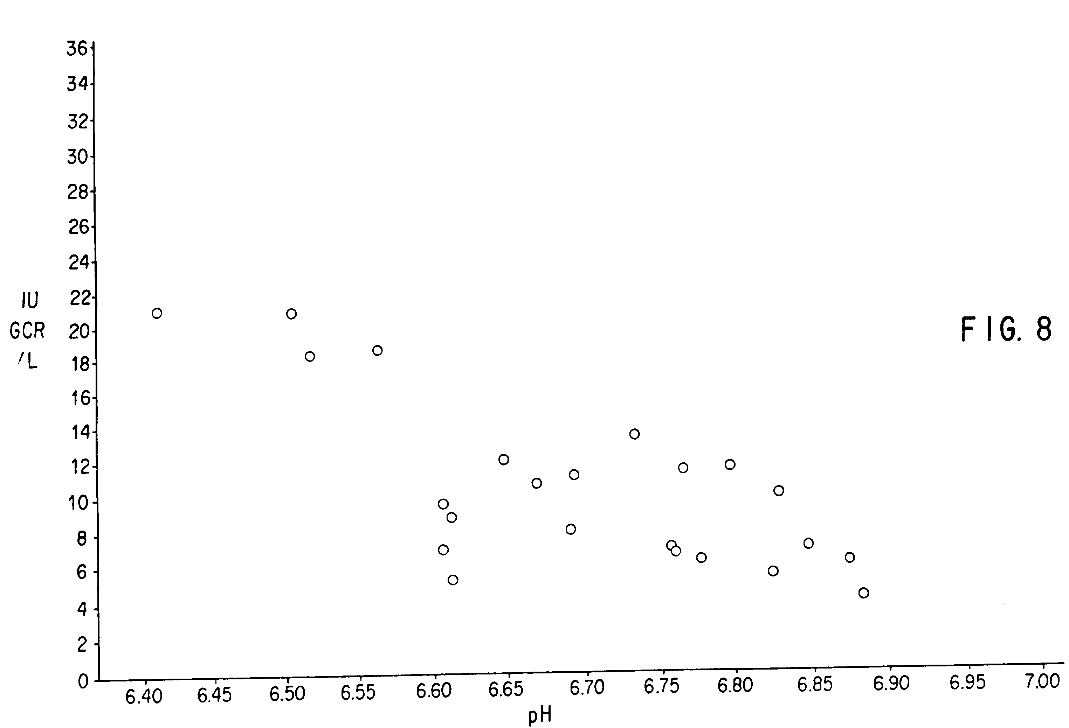

The level of active enzyme as a function of albumin concentration is shown in Fig. 7 and as a function of pH in Fig. 8. Selected samples were also analyzed by a polyclonal ELISA and demonstrated a constant level of GCR protein was being produced (1.41 ± 0.3 mg/l) indicating that the increased level of activity seen in the harvests was due to increased stability of the enzyme with the lower pH and higher level of albumin. The preferred pH was below 6.6.

### Example 5.

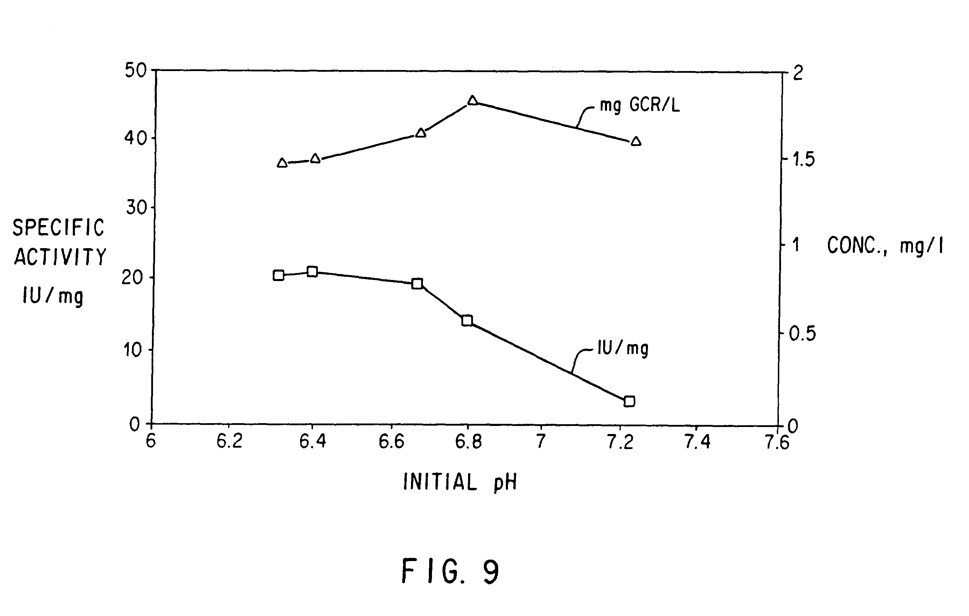

The II 15B cell line used in Example 1 was inoculated into t-25 plastic flasks at an initial density of 5 x 10⁵ cells/flask. The cells were overlaid with growth medium at pH 6.6 containing 10% FBS which was changed every 48 hours until the flasks were confluent. The growth medium was then removed and replaced with fresh growth medium which had been titrated to 5 different pH's ranging from 6.2 to 7.2. After 24 hours the medium was removed and assayed for the amount of GCR protein present by a polyclonal ELISA and the amount of GCR activity present by the 4-MU assay. The level of GCR protein produced was not affected by the pH (Fig. 9) but the specific activity was, resulting in a higher activity as the PH was decreased. The maximal activity was achieved with an initial pH of 6.2 - 6.7.

### Example 6.

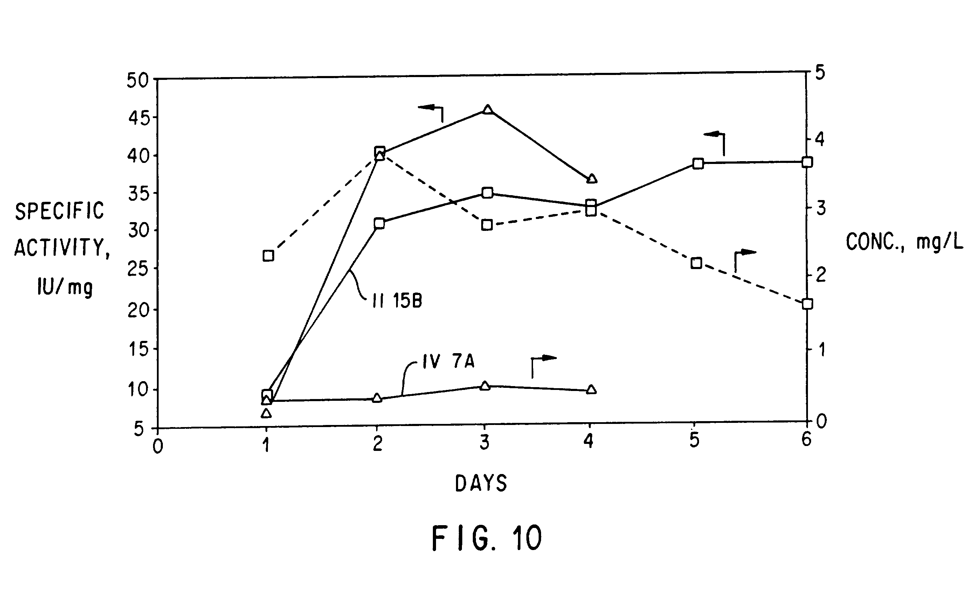

A second GCR expression vector was constructed as described in U.S. Patent Appl. 455,507, filed December 22, 1989 (Fig. 9) with transcription of the GCR gene under the control of a SV40 enhancer Adenovirus major late promoter. This vector was used to transfect DG44 CHO cells and GCR expressing cells were developed as in Example 1. One of the cell lines, named IV 7A, was inoculated into microcarrier culture as in Example 1 with growth medium containing 10% DCS. After a cell density of 5 X 10⁶ cells/ml was achieved, the cultures were placed in a production medium containing 1 mM DTT. Daily samples were taken for GCR analysis by the 4-MU assay and a polyclonal ELISA, and for analysis of pH and the concentration of dissolved oxygen. The conditioned medium (80%) was harvested daily and replaced with fresh medium as in Example 1. The pH was maintained below 6.6 and the dissolved oxygen was maintained below 0.06 mM. The productivity was compared to the II 15B cell line used for examples 1-3 (Fig. 10). Although the two cell lines produced different amounts of the enzyme (mg/l), the specific activity of the GCR was the same in the presence of 1 mM DTT, indicating that the activity of GCR in the extracellular medium was not dependent on the vector chosen or the level of GCR produced.

## Claims

1. A method of producing biologically active glucocerebrosidase comprising:
(i) culturing recombinant cells containing the DNA sequence encoding for the production of glucocerebrosidase in a growth medium suitable for cell growth;
(ii) replacing a substantial portion of the growth medium with a production medium that is suitable for cell viability and secretion and stabilization of glucocerebrosidase;
(iii) allowing production and secretion of glucocerebrosidase into the production medium;
(iv) removing and collecting the production medium containing the secreted glucocerebrosidase, obtained in step (iii); and
(v) purifying the glucocerebrosidase from the production medium collected in step (iv).

2. A method according to claim 1, wherein the step of removing and collecting the production medium is performed on a continuous basis and includes the step of continually replacing the production medium.

3. A method according to claim 2, wherein the production medium includes a thiol-containing composition.

4. A method according to claim 3, wherein the thiol-containing composition is selected from the group consisting of dithiothreitol, glutathione, 1,3-mercaptoglycerol, cysteamine, lipoic acid, lipoamide and thioglycolate.

5. A method according to claim 4, wherein the thiol-containing composition in the production medium is dithiothreitol and wherein the concentration of dithiothreitol in the production medium is in the range of 0.5-2 mM.

6. A method according to claim 2, wherein the production medium is maintained at a less than 0.06 mM concentration of molecular oxygen.

7. A method according to claim 2, wherein the pH of the production medium is adjusted to below 7.

8. A method according to claim 7, wherein the pH of the production medium is in the range of 6.2 - 6.7.

9. A method according to claim 2, wherein the production medium includes a protein.

10. A method for the production and stabilization of a protein comprising:
(i) culturing recombinant cells containing the DNA sequence encoding for the production of the protein in a growth medium suitable for cell growth;
(ii) replacing a substantial portion of the growth medium with a production medium, suitable for cell viability and secretion and stabilization of the protein, that includes dithiothreitol in the range of approximately 0.5-2 mM and is maintained at a less than 0.06 mM concentration of molecular oxygen and at a pH adjusted to below 7;
(iii) allowing production and secretion of the protein into the production medium;
(iv) removing and collecting the production medium containing the secreted protein, obtained in step (iii), on a continuous basis and continually replacing the production medium; and
(v) purifying the protein from the production medium collected in step (iv).

11. A method according to claim 10, wherein the pH of the production medium is in the range of 6.2 - 6.7.

12. A cell culture medium comprising:
(i) cells including means for secreting glucocerebrosidase; and
(ii) a composition that is not toxic to the cells and that stabilizes glucocerebrosidase.

13. A cell culture medium according to claim 12, wherein the composition includes a thiol-containing composition.

14. A cell culture medium according to claim 13, wherein the thiol-containing composition is selected from the group consisting of dithiothreitol, glutathione, 1,3-mercaptoglycerol, cysteamine, lipoic acid, lipoamide and thioglycolate.

15. A cell culture medium according to claim 14, wherein the thiol-containing composition is dithiothreitol and wherein the concentration of dithiothreitol in the culture medium is in the range of 0.5-2 mM.

16. A cell culture medium according to claim 12, wherein the cell culture medium is maintained at a less than 0.06 mM concentration of molecular oxygen.

17. A cell culture medium according to claim 12, wherein the pH is adjusted to below 7.

18. A cell culture medium according to claim 17, wherein the pH is in the range of 6.2-6.7.

19. A cell culture medium according to claim 12, wherein the composition includes a protein.

## Patentansprüche

1. Verfahren zur Gewinnung biologisch aktiver Glucocerebrosidase, umfassend:
(i) ein Kultivieren rekombinanter Zellen, welche die DNA-Sequenz enthalten, die kodiert für die Herstellung von Glucocerebrosidase, in einem zur Zellkultur geeigneten Kulturmedium;
(ii) ein Ersetzen eines erheblichen Teils des Kulturmediums durch ein Produktionsmedium, das die Zellebensfähigkeit sicherstellt sowie für die Sekretion und zur Stabilisierung von Glucocerebrosidase geeignet ist;
(iii) ein Produzieren- und Sezernieren-lassen von Glucocerebrosidase in das Produktionsmedium;
(iv) ein Entfernen und Sammeln des Produktionsmediums aus Schritt (iii), das die sezernierte Glucocerebrosidase enthält; und
(v) ein Reinigen der Glucocerebrosidase aus dem in Schritt (iv) gesammelten Produktionsmedium.

2. Verfahren nach Anspruch 1, wobei der Schritt des Entfernens und Sammelns des Produktionsmediums stetig erfolgt und den Schritt des stetigen Ersetzens des Produktionsmediums beinhaltet.

3. Verfahren nach Anspruch 2, wobei das Produktionsmedium eine Thiol-haltige Zusammensetzung enthält.

4. Verfahren nach Anspruch 3, wobei die Thiol-haltige Zusammensetzung ausgewählt ist aus der Gruppe Dithiothreitol, Glutathion, 1,3-Mercaptoglycerin, Cysteamin, Liponsäure, Liponsäureamid und Thioglycolat.

5. Verfahren nach Anspruch 4, wobei die Thiol-haltige Zusammensetzung in dem Produktionsmedium Dithiothreitol ist und die Konzentration von Dithiothreitol in dem Produktionsmedium von 0,5-2 mM reicht.

6. Verfahren nach Anspruch 2, wobei die Konzentration an molekularem Sauerstoff in dem Produktionsmedium auf unter 0,06 mM gehalten wird.

7. Verfahren nach Anspruch 2, wobei der pH-Wert des Produktionsmediums auf unter 7 eingestellt wird.

8. Verfahren nach Anspruch 7, wobei der pH-Wert des Produktionsmediums von 6,2-6,7 reicht.

9. Verfahren nach Anspruch 2, wobei das Produktionsmedium ein Protein beinhaltet.

10. Verfahren zur Herstellung und Stabilisierung eines Proteins, umfassend:
(i) Kultivieren rekombinanter Zellen, die die DNA-Sequenz enthalten, welche kodiert für die Herstellung des Proteins, in einem zum Zellwachstum geeigneten Kulturmedium;
(ii) Ersetzen eines erheblichen Teils des Kulturmediums durch ein Produktionsmedium, das die Zellebensfähigkeit sicherstellt und für die Sekretion sowie zur Stabilisierung des Proteins geeignet ist, das ungefähr 0,5-2 mM Dithiothreitol enthält und bei dem die Konzentration an molekularem Sauerstoff unter 0,06 mM gehalten und der pH-Wert auf unter 7 eingestellt wird;
(iii) Herstellen- und Sezernieren-lassen des Proteins in das Produktionsmedium;
(iv) Stetiges Entfernen und Sammeln des Produktionsmediums, welches das sezernierte Protein enthält und das in Schritt (iii) erhalten worden ist, sowie stetiges Ersetzen des Produktionsmediums; und
(v) Reinigen des Proteins aus dem in Schritt (iv) gesammelten Produktionsmedium.

11. Verfahren nach Anspruch 10, wobei der pH-Wert des Produktionsmediums von 6,2-6,7 reicht.

12. Zellkulturmedium, umfassend:
(i) Zellen, die Glucocerebrosidase sezernieren können; und
(ii) eine Zusammensetzung, die für die Zellen nicht toxisch ist und Glucocerebrosidase stabilisiert.

13. Zellkulturmedium nach Anspruch 12, wobei die Zusammensetzung eine Thiol-haltige Verbindung enthält.

14. Zellkulturmedium nach Anspruch 13, wobei die Thiol-haltige Verbindung ausgewählt ist aus der Gruppe Dithiothreitol, Glutathion, 1,3-Mercaptoglycerin, Cysteamin, Liponsäure, Liponsäureamid und Thioglycolat.

15. Zellkulturmedium nach Anspruch 14, wobei die Thiol-haltige Verbindung Dithiothreitol ist und die Konzentration von Dithiothreitol in dem Kulturmedium von 0,5-2 mM reicht.

16. Zellkulturmedium nach Anspruch 12, wobei die Konzentration an molekularem Sauerstoff in dem Zellkulturmedium auf unter 0,06 mM gehalten wird.

17. Zellkulturmedium nach Anspruch 12, wobei der pH-Wert auf unter 7 eingestellt wird.

18. Zellkulturmedium nach Anspruch 17, wobei der pH-Wert von 6,2-6,7 reicht.

19. Zellkulturmedium nach Anspruch 12, wobei die Zusammensetzung ein Protein beinhaltet.

## Revendications

1. Méthode de production de glucocérébrosidase biologiquement active comprenant :
(i) la culture de cellules recombinées contenant la séquence d'ADN codant pour la production de glucocérébrosidase dans un milieu de croissance convenant pour la culture de cellules ;
(ii) le remplacement d'une partie substantielle du milieu de croissance par un milieu de production approprié à la viabilité des cellules ainsi qu'à la sécrétion et à la stabilisation de la glucocérébrosidase ;
(iii) la production et la sécrétion de glucocérébrosidase dans le milieu de production ;
(iv) l'enlèvement et la récupération du moyen de production contenant la glucocérébrosidase sécrétée, obtenue à l'étape (iii) ; et
(v) la purification de la glucocérébrosidase à partir du milieu de production recueilli à l'étape (iv).

2. Méthode selon la revendication 1, dans laquelle l'étape d'enlèvement et de récupération du moyen de production s'effectue de façon continue et comporte une étape de remplacement en continu du milieu de production.

3. Méthode selon la revendication 2, dans laquelle le milieu de production contient une composition contenant un thiol.

4. Méthode selon la revendication 3, dans laquelle la composition contenant un thiol est choisie dans le groupe constitué par le dithio thréitol, le glutathione, le 1,3-mercaptoglycérol, la cystéamine, l'acide lipoïque, une lipoamine et un thioglycolate.

5. Méthode selon la revendication 4, dans laquelle la composition contenant un thiol dans le milieu de production est le dithiothréitol et dans laquelle la concentration en dithiothréitol dans le milieu de production est comprise entre 0,5 et 2 mM.

6. Méthode selon la revendication 2, dans laquelle la concentration en oxygène moléculaire dans le milieu de production est maintenue à une valeur inférieure à 0,06 mM.

7. Méthode selon la revendication 2, dans laquelle le pH du milieu de production est ajusté à une valeur inférieure à 7.

8. Méthode selon la revendication. 7, dans laquelle le pH du milieu de production se situe dans la plage de 6,2 à 6,7.

9. Méthode selon la revendication 2, dans laquelle le milieu de production inclut une protéine.

10. Méthode de production et de stabilisation d'une protéine comprenant :
(i) la culture de cellules recombinées contenant la séquence d'ADN qui code pour la production de la protéine dans un milieu de croissance convenant pour la culture de cellules ;
(ii) le remplacement d'une partie substantielle du milieu de croissance par un milieu de production approprié à la viabilité et à la sécrétion des cellules et à la stabilisation de la protéine, qui comporte du dithiothréitol dans la plage approximative de 0,5 à 2 mM, et dans lequel la concentration en oxygène moléculaire est maintenue à une valeur inférieure à 0,06 mM et le pH ajusté à une valeur inférieure à 7 ;
(iii) la production et la sécrétion de la protéine dans le milieu de production ;
(iv) l'enlèvement et la récupération du moyen de production contenant la protéine sécrétée, obtenue à l'étape (iii), de façon continue, et le remplacement en continu du milieu de production ; et
(v) la purification de la protéine à partir du milieu de production recueilli à l'étape (iv).

11. Méthode selon la revendication 10, dans laquelle le pH du milieu de production se situe dans la plage de 6,2 à 6,7.

12. Milieu de culture cellulaire comprenant :
(i) des cellules contenant un moyen de sécrétion de la glucocérébrosidase ; et
(ii) une composition qui n'est pas toxique pour les cellules et qui stabilise la glucocérébrosidase.

13. Milieu de culture cellulaire selon la revendication 12, dans lequel la composition inclut un composé contenant un thiol.

14. Milieu de culture cellulaire selon la revendication 13, dans lequel le composé contenant un thiol est choisi dans le groupe constitué par le dithiothréitol, le glutathione, le 1,3-mercaptoglycérol, la cystéamine, l'acide lipoïque, une lipoamine et un thioglycolate.

15. Milieu de culture cellulaire selon la revendication 14, dans lequel le composé contenant un thiol est le dithiothréitol et dans lequel la concentration en dithiothréitol dans le milieu de culture est comprise entre 0,5 et 2 mM.

16. Milieu de culture cellulaire selon la revendication 12, dans lequel la concentration en oxygène moléculaire du milieu de culture cellulaire est maintenue à une valeur inférieure à 0,06 mM.

17. Milieu de culture cellulaire selon la revendication 12, dans lequel le pH est ajusté à une valeur inférieure à 7.

18. Milieu de culture cellulaire selon la revendication 17, dans lequel le pH se situe dans la plage de 6,2 à 6,7.

19. Milieu de culture cellulaire selon la revendication 12, dans lequel la composition inclut une protéine.
